# FASCICULE DE BREVET EUROPEEN

(11) **EP 3 601 244 B1**
(45) Date de publication et mention de la délivrance du brevet: **19.05.2021**
(21) Numéro de dépôt: 18715217.8
(22) Date de dépôt: 21.03.2018
(51) Int. Cl.: C07D 295/084, H01M 10/0568, H01G 11/62

(54) **LIQUIDES IONIQUES, ÉLECTROLYTES ET DISPOSITIF DE STOCKAGE LES COMPRENANT**
IONISCHE FLÜSSIGKEITEN, ELEKTROLYTEN UND SPEICHERVORRICHTUNG DIE DIESE ENTHALTEN
IONIC LIQUIDS, ELECTROLYTES AND STORAGE DEVICE COMPRISING THEM

(30) Priorité: 23.03.2017 FR 1752436
(43) Date de publication de la demande: 05.02.2020
(73) Titulaire: Commissariat à l'Énergie Atomique et aux Énergies Alternatives, 75015 Paris (FR)
(72) Inventeur: CADRA, Stéphane, 37550 Saint Avertin (FR); SZYMCZAK, Jonathan, 29000 Quimper (FR); LE DIGABEL, Matthieu, 37260 Monts (FR); BILLER, Agnès, 37550 Saint-Avertin (FR)
(74) Mandataire: Brevalex
(86) Numéro de dépôt international: PCT/FR2018/050682
(87) Numéro de publication internationale: WO 2018/172696

(56) Documents cités:
- EP-A1- 1 380 569
- EP-A1- 1 642 894
- EP-A1- 1 837 333
- US-A1- 2012 028 168
- FERRARI S ET AL: "A binary ionic liquid system composed of N-methoxyethyl-N-methylpyrrolidinium bis(trifluoromethanesulfonyl)-imide and lithium bis(trifluoromethanesulfonyl)imide: A new promising electrolyte for lithium batteries", JOURNAL OF POWER SOURCES, ELSEVIER SA, CH, vol. 194, no. 1, 20 octobre 2009 (2009-10-20), pages 45-50, XP026446317, ISSN: 0378-7753 [extrait le 2008-12-09] cité dans la demande

## Description

### DOMAINE TECHNIQUE

La présente invention a trait à de nouveaux liquides ioniques résultant de l'association entre un cation spécifique et un anion spécifique, ces liquides ioniques présentant, notamment de bonnes propriétés de conductivité, et plus spécifiquement une conductivité pouvant être supérieure à 1 mS/cm. L'invention a trait également à de nouveaux sels utilisables comme liquides ioniques conformes à l'invention ou comme produits intermédiaires pour la conception de liquides ioniques conformes à l'invention.

Un sel est obtenu par association d'un composé anionique (*a fortiori,* chargé négativement) avec un composé cationique (*a fortiori,* chargé positivement).

Parmi les sels, les liquides ioniques sont des sels se présentant à l'état liquide à température ambiante (le point de fusion étant inférieur à 20°C), par opposition à des sels classiques, comme le chlorure de sodium, qui présentent un point de fusion proche de 180°C, ces liquides ioniques pouvant être représentés par la formule générale suivante :

A⁺X⁻

dans laquelle :
^{*}A⁺ représente un cation généralement organique; et
^{*}X⁻ représente un anion organique ou minéral.

La particularité des liquides ioniques en termes d'état provient, notamment, de la différence morphologique entre l'anion et le cation (par exemple, au niveau de l'encombrement stérique et de la géométrie) peu favorable à l'établissement d'une forme cristalline du sel.

En outre, les liquides ioniques présentent une faible toxicité, une très faible inflammabilité, une stabilité électrochimique et une conductivité ionique intéressante.

De ce fait, les liquides ioniques présentent un grand intérêt dans les domaines nécessitant la mise en œuvre de solutions conductrices d'ions et peuvent être, notamment, utilisés comme solvants de synthèse, solutions d'électrodéposition ou encore d'électrolytes de dispositifs à stockage d'énergie, tels que les batteries sécuritaires de dernière génération, comme les batteries lithium-soufre, les batteries lithium-ion ou encore les batteries à flux redox ou encore des dispositifs solaires, tels que les cellules solaires à pigment photosensible.

Toutefois, dans le domaine des batteries, le point limitant de ces liquides ioniques reste leur haute viscosité et leur incompatibilité vis-à-vis de certains matériaux d'électrodes, comme cela est le cas avec le graphite, ce qui induit une limitation des performances des batteries en terme de cyclage, le cyclage désignant communément le nombre de cycles de charge/décharge qui peut être effectué par une batterie.

Pour surmonter ces inconvénients, certains auteurs ont travaillé sur la modification des liquides ioniques, par exemple, en ajoutant des fonctionnalités particulières à la composante cationique et/ou anionique en vue d'améliorer les propriétés intrinsèques du sel et lui conférer une fonctionnalité particulière, par exemple, une viscosité plus faible.

En particulier, Ferrari et al., dans Journal of Power Sources, 194, 45-50, 2009 et Wu et al., dans Electrochimica Acta, 184, 356-363, 2015 décrivent des liquides ioniques comprenant des cations pyrrolidinium dont les groupes alkyles ont été remplacés par des groupes alcoxy, les liquides ioniques résultant pouvant présenter une réduction de viscosité de 12% par rapport à leurs homologues alkyles.

Au vu de ce qui existe déjà, les auteurs de la présente invention ont mis au point de nouveaux liquides ioniques qui présentent, notamment, une conductivité importante (au moins supérieure à 1 mS/cm) et une viscosité moindre compatible avec une utilisation de ces liquides ioniques comme électrolytes et ont mis au point également de nouveaux sels pouvant être utilisés comme liquides ioniques conformes à l'invention ou comme sels intermédiaires pour la fabrication de ces liquides ioniques.

### EXPOSÉ DE L'INVENTION

Ces nouveaux liquides ioniques comprennent ainsi l'association d'un cation répondant à la formule (I) suivante : 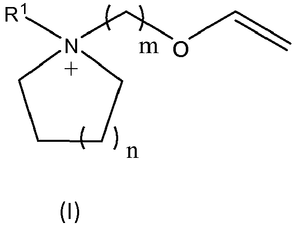 dans laquelle :
- R¹ est un groupe hydrocarboné acyclique ;
- n est un entier allant de 0 à 3 ;
- m est un entier allant de 1 à 4 ;
et d'un anion choisi parmi un anion nitrate, un anion phosphate ou un anion imidure.

Il s'entend que le cation de formule (I) et les anions mentionnés ci-dessus sont associés de sorte à assurer l'électroneutralité du liquide ionique résultant (en d'autres termes, un liquide ionique dont la ou les charges positives du ou desdits cations équilibrent la ou les charges négative du ou desdits anions).

De manière plus explicite, le cation de formule (I) peut correspondre, selon les valeurs de n, à l'une des formules suivantes :
^{*}pour n=0, la formule (la) suivante : 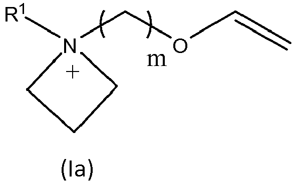
^{*}pour n=1, la formule (Ib) suivante : 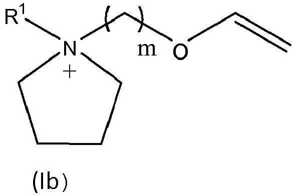
^{*}pour n=2, la formule (Ic) suivante : 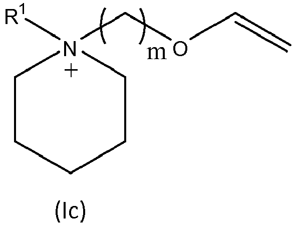
^{*}pour n=3, la formule (Id) suivante :

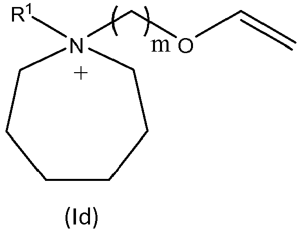

De manière avantageuse, les liquides ioniques de l'invention sont des liquides ioniques, dans lesquels le cation est un cation de formule (I) avec n étant égal à 1 (soit, en d'autres termes, un cation de formule (Ib)).

Le groupe R¹ est un groupe hydrocarboné acyclique et, plus spécifiquement il peut s'agir d'un groupe hydrocarboné acyclique, linéaire ou ramifié, tel qu'un groupe alkyle, comportant de 1 à 4 atomes de carbone. Encore plus spécifiquement, le groupe R¹ peut être un groupe de formule -CₚH₂ₚ₊₁, avec p étant un entier allant de 1 à 4, par exemple, un groupe méthyle, un groupe éthyle, un groupe n-propyle, un groupe isopropyle, un groupe n-butyle, un groupe isobutyle, un groupe tertiobutyle.

A titre d'exemple, R¹ peut être un groupe méthyle.

A titre d'exemple, m peut être égal à 2.

Concernant les anions :
- lorsque l'anion est un anion nitrate, celui-ci répond à la formule NO₃⁻ ;
- lorsque l'anion est un anion phosphate, celui-ci répond à la formule PO₄³⁻ ;
- lorsque l'anion est un anion imidure, cela signifie, classiquement, qu'il comporte un radical imide, dont la charge négative est portée par l'atome d'azote, lequel atome d'azote est lié à deux groupes carbonyles ou deux groupes sulfonyles, ledit radical imide pouvant être représenté par l'une des formules (II) et (III) suivantes : 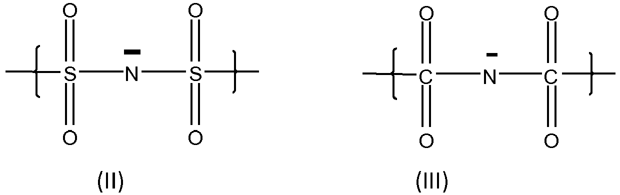
les accolades indiquant que les groupes -SO₂- et -CO₂- sont liés à d'autres groupes.

Avantageusement, l'anion est un anion imidure, dont la charge négative est portée par l'atome d'azote, lequel atome d'azote est lié à deux groupes sulfonyles, un tel anion pouvant être représenté par la formule générale (II') suivante : 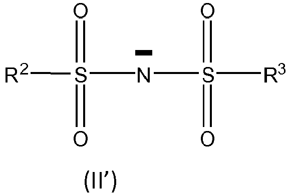 dans laquelle R² et R³ représentent, indépendamment l'un de l'autre, un atome de fluor ou un groupe perfluorocarboné.

Plus spécifiquement, R² et R³ peuvent représenter, tous deux, un atome de fluor ou, tous deux, un groupe perfluorocarboné, par exemple, un groupe perfluorométhyle -CF₃.

Des anions imidures particuliers répondant à ces spécificités sont ceux de formules (IV) et (V) suivantes : 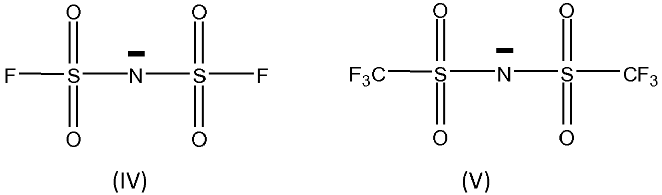 connus également sous le nom de *bis*(fluorosulfonyl)imidure et de *bis*(trifluorométhanesulfonyl)imidure.

Des liquides ioniques, conformes à l'invention sont, avantageusement, constitués par l'association d'un cation de formule (Ib) et d'un anion répondant à l'une des formules (IV) ou (V) susmentionnées.

Plus spécifiquement, pour le cation de formule (Ib), R¹ peut être un groupe méthyle et m peut être égal à 2, auquel cas le cation répond à la formule (IIb) suivante : 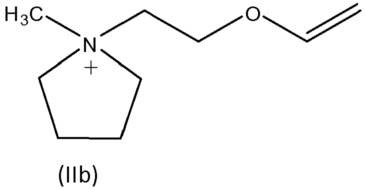 ce cation pouvant être dénommé N-(méthyl)-(2-vinyloxyéthyl)pyrrolidinium.

Des liquides ioniques conformes à l'invention sont des liquides ioniques résultant de l'association d'un cation de formule (IIb) et d'un anion de formule (IV) ou (V), lesquels liquides ioniques répondant ainsi aux formules respectives (VI) et (VII) suivantes : 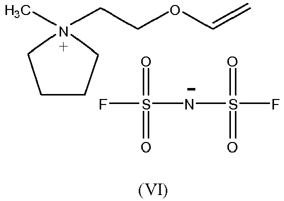 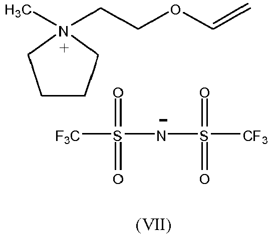 ces liquides ioniques pouvant être nommés respectivement *bis*(fluorosulfonyl)imidure de N-(méthyl)-(2-vinyloxyéthyl)pyrrolidinium et *bis*(trifluorométhanesulfonyl)imidure de N-(méthyl)-(2-vinyloxyéthyl)pyrrolidinium.

L'invention a également trait à une nouvelle famille de sels, dont certains constituent des liquides ioniques tels que définis ci-dessus ou peuvent être utilisés comme sels intermédiaires pour la fabrication de liquides ioniques conformes à l'invention.

Ces sels conformes à l'invention comprennent l'association d'au moins un cation répondant à la formule (Ib) suivante : 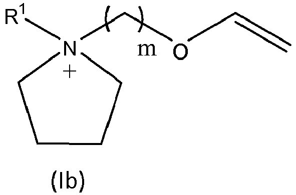 dans laquelle :
- R¹ est un groupe hydrocarboné acyclique ;
- m est un entier allant de 1 à 4 ;
et d'au moins un anion Y.

Les déclinaisons mentionnées ci-dessus au sujet des cations de formule (I) pour les liquides ioniques sont également valables pour ces sels (notamment en ce qui concerne R¹ et m).

Il s'entend que le ou les cations de formule (Ib) et le ou les anions Y sont associés de sorte à assurer l'électroneutralité du sel résultant (en d'autres termes, un sel dont la ou les charges positives du ou desdits cations équilibrent la ou les charges négatives du ou desdits anions).

L'anion Y peut être un anion (en d'autres termes, le contre-ion associé au cation de formule (I)) choisi parmi les anions halogénures (par exemple, chlorure, bromure ou iodure), un anion nitrate, un anion phosphate, les anions imidures. Par exemple, l'anion Y est un anion halogénure du type chlorure.

Dans le cas où l'anion Y est un anion nitrate, un anion phosphate ou un anion imidure, les sels résultants constituent une classe spécifique de liquides ioniques conformes à l'invention.

Les liquides ioniques, conformes à l'invention, peuvent être utilisés seuls ou en mélange avec un sel différent de ceux conformes à l'invention, lequel sel peut être un sel de lithium ou un sel de potassium, lesdits liquides ioniques pouvant être utilisés comme électrolytes, en particulier, des électrolytes pour dispositifs de stockage d'énergie, tels que des batteries lithium-ion, des batteries lithium-soufre, les batteries à flux redox ou encore les supercondensateurs.

Aussi, l'invention a également trait à un électrolyte comprenant au moins un liquide ionique tel que défini ci-dessus.

L'électrolyte peut comprendre, en outre, au moins un sel de lithium ou au moins un sel de potassium.

Selon un mode particulier de l'invention, l'électrolyte peut être constitué uniquement d'au moins un liquide ionique tel que défini ci-dessus ou peut comprendre, en outre, au moins un sel de lithium ou d'au moins un sel de potassium.

A titre d'exemples de sel de lithium, on peut citer l'hexafluorophosphate de lithium (LiPF₆), le bis(oxalatoborate) de lithium, le tétrafluoroborate de lithium (LiBF₄), le *bis*(trifluorométhanesulfonyl)imidure de lithium (connu sous l'abréviation LiTFSI), le *bis*(fluorosulfonyl)imidure de lithium, l'hexafluoroarsénate de lithium (LiAsF₆), le nitrate de lithium (LiNO₃) ou encore le perchlorate de lithium (LiClO₄).

A titre d'exemples de sel de potassium, on peut citer l'hexafluorophosphate de potassium (KPF₆), le tétrafluoroborate de potassium (KBF₄), le *bis*(trifluorométhanesulfonyl)imidure de potassium, le *bis*(fluorosulfonyl)imidure de potassium, l'hexafluoroarsénate de potassium (KAsF₆), le nitrate de potassium (KNO₃) ou encore le perchlorate de potassium (KClO₄).

Le sel de lithium ou le sel de potassium peut être compris, dans l'électrolyte, à une concentration n'excédant pas 1,5 mole de sel par litre de liquide ionique.

Les sels conformes à l'invention et les liquides ioniques conformes à l'invention peuvent être préparées par tous types de méthodes à la portée de l'homme du métier, telles que des méthodes impliquant une substitution ou un échange ionique.

A titre d'exemples, lorsque les sels conformes à l'invention comportent, comme anion, un anion halogénure, la préparation peut consister à faire réagir un composé de formule (VIII) suivante : 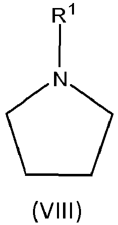 avec R¹ étant tel que défini ci-dessus ;
avec un composé de formule (IX) suivante : 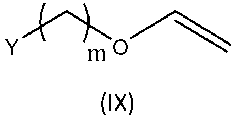 avec m étant tel que défini ci-dessus et Y correspondant à un atome d'halogène.

Sans être lié par la théorie, la réaction du composé de formule (VIII) sur le composé de formule (IX) consiste en une substitution nucléophile engendrant un groupe partant Y, qui constitue ainsi un anion halogénure.

A titre d'exemples, lorsque les liquides ioniques conformes à l'invention comportent, comme anion, un anion imidure, un anion nitrate ou un anion phosphate, la préparation peut consister en un échange ionique entre un sel consistant en l'association d'un cation de formule (I) susmentionnée et d'un anion halogénure (les sels pour lesquels le cation est de formule (Ib) étant des sels conformes à l'invention) et un sel consistant en l'association d'un cation, par exemple, alcalin (tel que du lithium, du sodium ou du potassium) et d'un anion imidure, d'un anion nitrate ou d'un anion phosphate (ce sel étant nommé, par après, sel secondaire).

L'échange ionique est réalisé typiquement en milieu aqueux et se caractérise par la formation de deux phases : une phase dite organique comprenant le sel comprenant l'association d'un cation de formule (I) et d'un anion imidure, nitrate ou phosphate et une phase aqueuse comprenant l'association d'un anion halogénure et d'un cation, par exemple, alcalin (issu du sel secondaire).

La phase aqueuse est éliminée par décantation et la phase organique subit un traitement pouvant passer par une opération d'extraction à l'aide d'un solvant organique, la nouvelle phase organique résultante pouvant être ensuite soumise à une filtration sur charbon actif, un séchage sous vide d'air et à une déshydratation sur zéolite. La déshydratation peut être poussée jusqu'à obtenir une quantité d'eau dans le liquide ionique inférieure à 50 ppm.

Comme déjà mentionné ci-dessus, les liquides ioniques de l'invention ainsi que les électrolytes définis plus haut présentent un intérêt important dans le domaine du stockage électrochimique justifié notamment, par exemple, en raison de leur faible inflammabilité (qui est une propriété intrinsèque liée au liquides ioniques), d'une conductivité élevée (telle qu'une conductivité supérieure à 2 mS/cm) et de leur température de fusion inférieure ou égale à -20°C.

Eu égard aux propriétés susmentionnées les compositions comprenant des liquides ioniques conformes à l'invention peuvent être utilisés comme électrolytes, en particulier, dans un dispositif à stockage d'énergie, par exemple, un accumulateur au lithium.

L'invention a donc trait également à un dispositif à stockage comprenant au moins une cellule comprenant une électrode positive et une électrode négative séparées l'une de l'autre par un séparateur comprenant, un électrolyte conforme à l'invention. En outre, la cellule peut être reliée à un réservoir, qui permet, *via* une pompe, l'acheminement de l'électrolyte conforme à l'invention au niveau du séparateur. La cellule est également reliée à un chargeur pour effectuer les opérations de charge.

L'électrode positive peut être à base d'un matériau carboné et l'électrode négative peut être à base d'un matériau lithié, par exemple, un matériau à base d'oxyde(s) métallique(s), tel que Li₄Ti₅O₁₂.

L'invention va à présent être décrite en référence aux exemples fournis ci-dessous donnés à titre illustratif et non limitatif.

### EXPOSÉ DÉTAILLÉ DE MODES DE RÉALISATION PARTICULIERS

### EXEMPLE 1

Cet exemple illustre la préparation d'un sel conforme à l'invention : le chlorure de N-(méthyl)-(2-vinyloxyéthyl)pyrrolidinium de formule suivante :

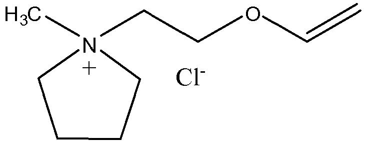

Pour ce faire, 25 g de 2-chlorovinyloxyéthyle (0,234 mol) et 25 g de méthylpyrrodiline (0,293 mol) sont préalablement distillés puis dilués dans 200 mL d'acétonitrile. Le mélange est maintenu sous agitation pendant 120 heures à 30°C. Les réactifs en excès et le solvant sont ensuite évaporés sous pression réduite.

A l'issue de cette évaporation, on obtient environ 14 g d'un liquide de couleur jaune-orange (0,073 mol), soit un rendement d'environ 30%.

Le liquide obtenu est analysé par RMN ¹H et RMN ¹³C, dont les résultats sont reportés ci-dessous.

RMN ¹H (D₂O)= 2,16 (br s, 4H) ; 3,02 (s, 3H) ; 3,67-3,49 (m, 6H) ; 4,35-4,17 (m, 4H) ; 6,2 (m, 1H).

RMN ¹³C (D₂O)= 21,1 (CH₂); 48,2 (CH₃); 62,2 (CH₂); 62,4 (CH₂); 65,3 (CH₂); 89,0 (CH₂); 150,1 (CH).

Ces résultats attestent que le produit obtenu est le sel répondant à la formule définie ci-dessus.

### EXEMPLE 2

Cet exemple illustre la préparation d'un liquide ionique conforme à l'invention : le *bis*(fluorosulfonyl)imidure de N-(méthyl)-(2-vinyloxyéthyl)pyrrolidinium de formule suivante :

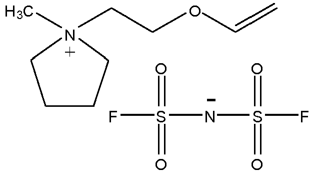

Pour ce faire, 19 g (0,099 mol) du sel de l'exemple 1 et 22 g de *bis*(fluorosulfonyl)imidure de potassium (0,100 mL) sont dissous respectivement dans 100 mL d'eau ultrapure (présentant une résistivité de 18,2 mΩ.cm⁻¹) pour former deux solutions (respectivement, une solution comprenant le sel de l'exemple 1 et une solution comprenant le *bis*(fluorosulfonyl)imidure de potassium). Les deux solutions sont mélangées à température ambiante pendant 24 heures. De ce mélange résultent une phase aqueuse comprenant du chlorure de potassium et les réactifs en excès et une phase organique comprenant essentiellement du *bis*(fluorosulfonyl)imidure de N-(méthyl)-(2-vinyloxyéthyl)pyrrolidinium. La phase organique est récupérée à l'aide de dichlorométhane puis transférée dans une ampoule à décanter, afin d'être lavée 5 fois avec 100 mL d'eau ultrapure. La phase organique est isolée puis le dichlorométhane est éliminé par évaporation sous pression réduite, afin d'obtenir un liquide ionique brut. Ce liquide ionique est ensuite dilué dans de l'acétate d'éthyle puis du charbon actif (13 g) est ajouté. Le mélange est placé sous agitation pendant 96 heures à 35°C. Le charbon actif est ensuite éliminé par filtration. La solution est ensuite purifiée par ajout d'environ 25±5 g d'alumine puis le mélange résultant est agité pendant au moins 5 heures à température ambiante. L'alumine est ensuite éliminée par filtration puis le liquide ionique est récupéré après élimination de l'acétate d'éthyle par évaporation sous vide (35°C, 1 mbar) pendant 72 heures.

A l'issue de cette élimination, on obtient environ 24 g (soit 0,070 mol) d'un liquide de couleur jaune pâle.

Le liquide obtenu est analysé par RMN ¹H et RMN ¹³C, dont les résultats sont reportés ci-dessous.

RMN ¹H (CDCl₃)= 2,10 (br s, 4H) ; 2,90 (s, 3H) ; 3,55-3,39 (m, 6H) ; 4,22-3,94 (m, 4H) ; 6,32 (m, 1H).

RMN ¹³C (CDCl₃)= 21,0 (CH₂); 48,4 (CH₃); 62,1 (CH₂); 62,3 (CH₂); 65,4 (CH₂); 88,9 (CH₂); 150,2 (CH).

Ces résultats attestent que le produit obtenu est le liquide ionique répondant à la formule définie ci-dessus.

### EXEMPLE 3

Cet exemple illustre la préparation d'un liquide ionique conforme à l'invention : le *bis*(trifluorométhanesulfonyl)imidure de N-(méthyl)-(2-vinyloxyéthyl)pyrrolidinium de formule suivante :

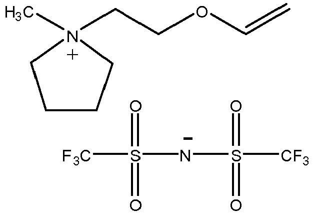

Le protocole de synthèse est similaire à celui décrit dans le cadre de l'exemple 2, si ce n'est que 14 g de chlorure de N-(méthyl)-(2-vinyloxyéthyl)pyrrolidinium (0,073 mol) ont été utilisés et 21 g de *bis*(trifluorométhanesulfonyl)imidure de lithium (0,073 mol) ont été utilisés en lieu et place des 22 g de *bis*(fluorosulfonyl)imidure de potassium.

Il est obtenu environ 22 g d'un liquide ionique jaune pâle.

Le liquide obtenu est analysé par RMN ¹H et RMN ¹³C, dont les résultats sont reportés ci-dessous.

RMN ¹H (CDCl₃)= 2,17 (br s, 4H) ; 3,02 (s, 3H) ; 3,55-3,35 (m, 6H) ; 4,55-4,02 (m, 4H) ; 6,20 (m, 1H)

RMN ¹³C (CDCl₃)= 21,1 (CH₂); 48,4 (CH₃); 62,3 (CH₂); 62,5 (CH₂); 65,54 (CH₂); 89,0 (CH₂); 120 (q, CF₃); 150,3 (CH)

Ces résultats attestent que le produit obtenu est le liquide ionique répondant à la formule définie ci-dessus.

### EXEMPLE 4

Dans cet exemple, les liquides ioniques obtenus aux exemples 2 et 3 sont caractérisés en termes de conductivité (mS/cm) à 25°C, de viscosité (mPa.s) à 25°C et de température de cristallisation (°C).

Les résultats sont reportés dans le tableau ci-dessous.

| Liquide ionique | Conductivité (mS/cm) | Viscosité (mPa.s) | Température de cristallisation (°C) |
|---|---|---|---|
| Exemple 2 | 4,7 | 79,2 | -65 |
| Exemple 3 | 2,5 | 100,8 | -67 |

Les liquides ioniques présentent des valeurs de conductivité supérieures à 2 mS/cm et des températures de fusion inférieures à -20°C. Ces liquides ioniques présentent, de ce fait, un intérêt tout particulier pour une utilisation dans des systèmes de stockage électrochimique.

## Revendications

1. Liquide ionique comprenant l'association d'un cation répondant à la formule (I) suivante : 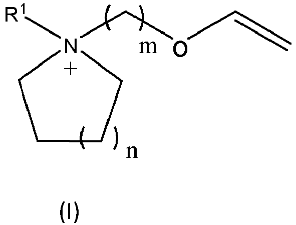 dans laquelle :
- R¹ est un groupe hydrocarboné acyclique ;
- n est un entier allant de 0 à 3 ;
- m est un entier allant de 1 à 4 ;
et d'un anion choisi parmi un anion nitrate, un anion phosphate ou un anion imidure.

2. Liquide ionique selon la revendication 1, dans lequel le cation de formule (I) répond à la formule spécifique (Ib) suivante : 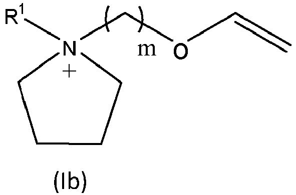 dans laquelle R¹ et m sont tels que définis à la revendication 1.

3. Liquide ionique selon la revendication 1 ou 2, dans lequel R¹ est un groupe alkyle comprenant de 1 à 4 atomes de carbone.

4. Liquide ionique selon l'une quelconque des revendications précédentes, dans lequel m est égal à 2.

5. Liquide ionique selon l'une quelconque des revendications précédentes, dans lequel l'anion est un anion imidure.

6. Liquide ionique selon l'une quelconque des revendications précédentes, dans lequel l'anion est un anion imidure répondant à la formule (II') suivante : 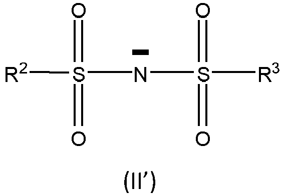 dans laquelle R² et R³ représentent, indépendamment l'un de l'autre, un atome de fluor ou un groupe perfluorocarboné.

7. Liquide ionique selon l'une quelconque des revendications précédentes, dans lequel l'anion est un anion imidure qui répond à l'une des formules (IV) et (V) suivantes :

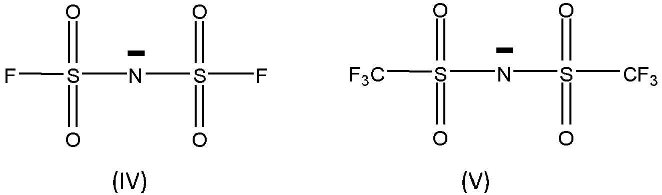

8. Liquide ionique selon l'une quelconque des revendications précédentes, qui répond à l'une des formules (VI) et (VII) suivantes :

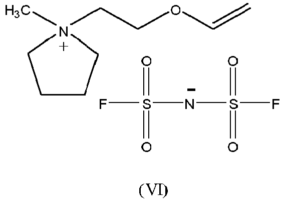

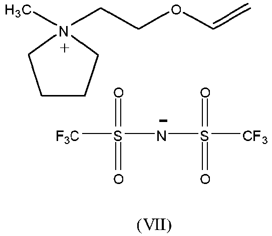

9. Sel comprenant l'association d'au moins un cation répondant à la formule (Ib) suivante : 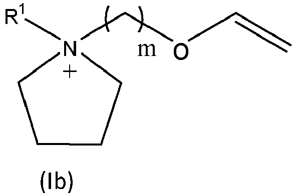 dans laquelle :
- R¹ est un groupe hydrocarboné acyclique ;
- m est un entier allant de 1 à 4 ;
et d'au moins un anion Y.

10. Sel selon la revendication 9, dans lequel l'anion Y est choisi parmi les anions halogénures, un anion nitrate, un anion phosphate, les anions imidures.

11. Electrolyte comprenant au moins un liquide ionique tel que défini selon l'une quelconque des revendications 1 à 8.

12. Electrolyte selon la revendication 11, comprenant, en outre, au moins un sel de lithium ou au moins un sel de potassium.

13. Electrolyte selon la revendication 12, dans lequel le sel de lithium est choisi parmi l'hexafluorophosphate de lithium (LiPF₆), le *bis*(oxalatoborate) de lithium, le tétrafluoroborate de lithium (LiBF₄), le *bis*(trifluorométhanesulfonyl)imidure de lithium (connu sous l'abréviation LiTFSI), le *bis*(fluorosulfonyl)imidure de lithium, l'hexafluoroarsénate de lithium (LiAsF₆), le nitrate de lithium (LiNO₃) ou encore le perchlorate de lithium (LiClO₄).

14. Electrolyte selon la revendication 12, dans lequel le sel de potassium est choisi parmi l'hexafluorophosphate de potassium (KPF₆), le tétrafluoroborate de potassium (KBF₄), le *bis*(trifluorométhanesulfonyl)imidure de potassium, le *bis*(fluorosulfonyl)imidure de potassium, l'hexafluoroarsénate de potassium (KAsF₆), le nitrate de potassium (KNO₃) ou encore le perchlorate de potassium (KClO₄).

15. Dispositif à stockage d'énergie comprenant au moins une cellule comprenant une électrode positive et une électrode négative séparées l'une de l'autre par un séparateur comprenant un électrolyte tel que défini selon l'une quelconque des revendications 11 à 14.

## Patentansprüche

1. Ionische Flüssigkeit, umfassend die Verbindung eines Kations, das der nachfolgenden Formel (I) genügt: 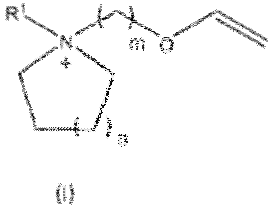 bei der:
- R¹ eine Acylkohlenwasserstoffgruppe ist;
- n eine ganze Zahl ist, die von 0 bis 3 geht;
- m eine ganze Zahl ist, die von 1 bis 4 geht;
und eines Anions, ausgewählt aus einem Nitratanion, einem Phosphatanion oder einem Imidanion.

2. Ionische Flüssigkeit nach Anspruch 1, bei der das Kation der Formel (I) der nachfolgenden speziellen Formel (Ib) genügt: 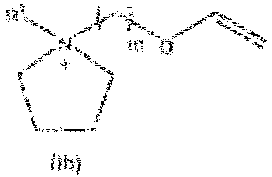 wobei R¹ und m wie in Anspruch 1 definiert sind.

3. Ionische Flüssigkeit nach Anspruch 1 oder 2, bei der R¹ eine Alkylgruppe ist, die von 1 bis 4 Kohlenstoffatome umfasst.

4. Ionische Flüssigkeit nach einem der vorhergehenden Ansprüche, bei der m gleich 2 ist.

5. Ionische Flüssigkeit nach einem der vorhergehenden Ansprüche, bei der das Anion ein Imidanion ist.

6. Ionische Flüssigkeit nach einem der vorhergehenden Ansprüche, bei der das Anion ein Imidanion ist, das der nachfolgenden Formel (11') genügt: 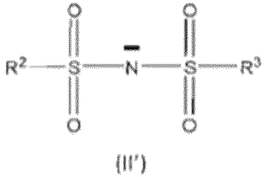 wobei R² und R³ unabhängig voneinander ein Fluoratom oder eine Perfluorkohlenstoffgruppe repräsentieren.

7. Ionische Flüssigkeit nach einem der vorhergehenden Ansprüche, bei der das Anion ein Imidanion ist, das einer der nachfolgenden Formeln (IV) und (V) genügt:

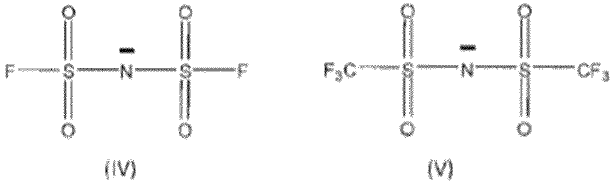

8. Ionische Flüssigkeit nach einem der vorhergehenden Ansprüche, die einer der nachfolgenden Formeln (VI) und (VII) genügt:

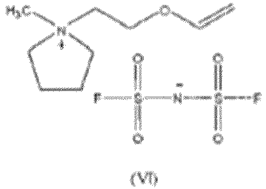

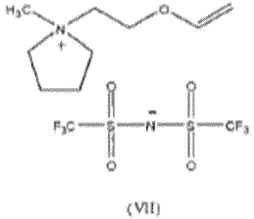

9. Salz, umfassend die Verbindung wenigstens eines Kations, das der nachfolgenden Formel (Ib) genügt: 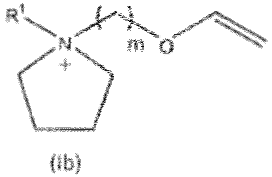 wobei:
- R¹ eine Acylkohlenwasserstoffgruppe ist;
- m eine ganze Zahl ist, die von 1 bis 4 geht;
und wenigstens eines Anions Y.

10. Salz nach Anspruch 9, bei dem das Anion Y ausgewählt ist aus den Halogenidanionen, einem Nitratanion, einem Phosphatanion, den Imidanionen.

11. Elektrolyt, umfassend wenigstens eine ionische Flüssigkeit wie nach einem der Ansprüche 1 bis 8 definiert.

12. Elektrolyt nach Anspruch 11, ferner umfassend wenigstens ein Lithiumsalz oder wenigstens ein Kaliumsalz.

13. Elektrolyt nach Anspruch 12, bei dem das Lithiumsalz ausgewählt ist aus Lithiumhexafluorphosphat (LiPF₆), Lithiumbis(oxalatborat), Lithiumtetrafluorborat (LiBF₄), Lithiumbis(trifluormethansulfonyl)imid (bekannt unter der Abkürzung LiTFSI), Lithiumbis(fluorsulfonyl)imid, Lithiumhexafluorarsenat (LiAsF₆), Lithiumnitrat (LiNO₃) oder aber Lithiumperchlorat (LiClO₄).

14. Elektrolyt nach Anspruch 12, bei dem das Kaliumsalz ausgewählt ist aus Kaliumhexafluorphosphat (KPF₆), Kaliumtetrafluorborat (KBF₄), Kaliumbis(trifluormethansulfonyl)imid, Kaliumbis(fluorsulfonyl)imid, Kaliumhexafluorarsenat (KAsF₆), Kaliumnitrat (KNO₃) oder aber Kaliumperchlorat (KClO₄).

15. Energiespeichervorrichtung, umfassend wenigstens eine Zelle, die eine positive Elektrode und eine negative Elektrode umfasst, die voneinander mittels eines Separators separiert sind, der einen Elektrolyt wie nach einem der Ansprüche 11 bis 14 definiert umfasst.

## Claims

1. An ionic liquid comprising the association of a cation having the following formula (I): 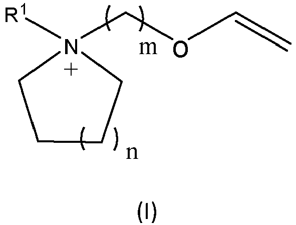 in which:
- R¹ is an acyclic hydrocarbon group;
- n is an integer ranging from 0 to 3;
- m is an integer ranging from 1 to 4;
and an anion chosen from a nitrate anion, a phosphate anion or an imide anion.

2. The ionic liquid according to claim 1, wherein the cation of formula (I) has the following specific formula (lb): 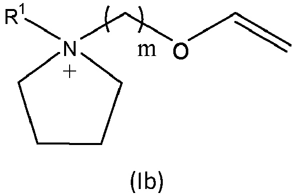 in which R¹ and m are as defined in claim 1.

3. The ionic liquid according to claim 1 or 2, wherein R¹ is an alkyl group comprising from 1 to 4 carbon atoms.

4. The ionic liquid according to any of the preceding claims, wherein m is 2.

5. The ionic liquid according to any of the preceding claims, wherein the anion is an imide anion.

6. The ionic liquid according to any of the preceding claims, wherein the anion is an imide anion having the following formula (II'): 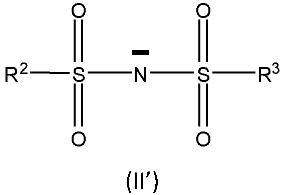 in which R² and R³ represent, independently of each other, a fluorine atom or a perfluorocarbon group.

7. The ionic liquid according to any of the preceding claims, wherein the anion is an imide anion which has one of the following formulae (IV) and (V):

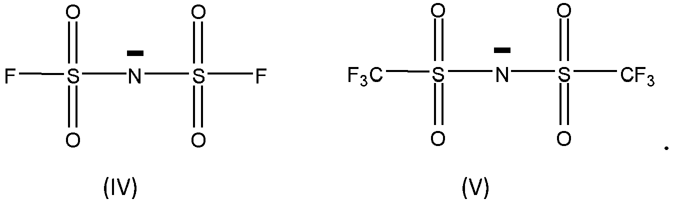

8. The ionic liquid according to any of the preceding claims, which has one of the following formulae (VI) and (VII):

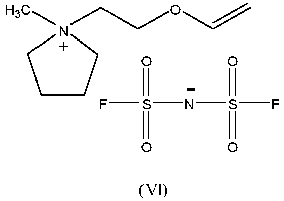

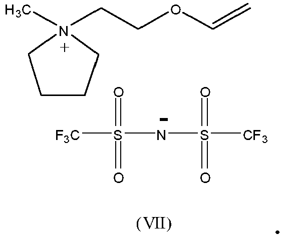

9. A salt comprising the association of at least one cation having the following formula (Ib): 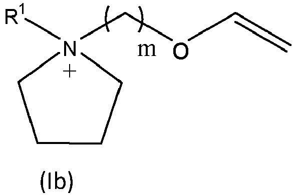 in which:
- R¹ is an acyclic hydrocarbon group;
- m is an integer ranging from 1 to 4;
and at least one anion Y.

10. The salt according to claim 9, wherein the anion Y is chosen from halide anions, a nitrate anion, a phosphate anion, imide anions.

11. An electrolyte comprising at least one ionic liquid as defined in any of claims 1 to 8.

12. The electrolyte according to claim 11, further comprising at least one lithium salt or at least one potassium salt.

13. The electrolyte according to claim 12, wherein the lithium salt is chosen from lithium hexafluorophosphate (LiPF₆), lithium *bis*(oxalatoborate), lithium tetrafluoroborate (LiBF₄), lithium bis(trifluoromethanesulphonyl)imide (known under the abbreviation LiTFSI), lithium bis(fluorosulphonyl)imide, lithium hexafluoroarsenate (LiAsF₆), lithium nitrate (LiNO₃) or even lithium perchlorate (LiClO₄).

14. The electrolyte according to claim 12, wherein the potassium salt is chosen from potassium hexafluorophosphate (KPF₆), potassium tetrafluoroborate (KBF₄), potassium *bis*(trifluoromethanesulphonyl)imide, potassium *bis*(fluorosulphonyl)imide, potassium hexafluoroarsenate (KAsF₆), potassium nitrate (KNO₃) or even potassium perchlorate (KClO₄).

15. An energy storage device comprising at least one cell comprising a positive electrode and a negative electrode which are separated from each other by a separator comprising an electrolyte as defined in any of claims 11 to 14.
